# EUROPEAN PATENT SPECIFICATION

(11) **EP 3 407 873 B1**
(45) Date of publication and mention of the grant of the patent: **21.08.2024**
(21) Application number: 17704661.2
(22) Date of filing: 30.01.2017
(51) Int. Cl.: A61K 9/16, A61K 31/225

(54) **PROCESS FOR PREPARATION OF A GRANULATE COMPRISING DIMETHYL FUMARATE**
HERSTELLUNGSVERFAHREN VON DIMETHYLFUMARATEGRANULAT
PROCÈS POUR LA FORMATION DES PARTICULES DE DIMÉTHYLFUMARATE

(30) Priority: 28.01.2016 EP 16460004
(43) Date of publication of application: 05.12.2018
(73) Proprietor: Zaklady Farmaceutyczne Polpharma S.A., 83-200 Starogard Gdanski (PL)
(72) Inventor: DROZD, Aleksandra, 55-003 Chrzastawa Mala (PL); SKOCZEN, Przemyslaw, 83-010 Straszyn (PL); CICHOCKI, Marek, 31-416 Krakow (PL)
(74) Representative: Chmurzynski, Sedzimir Lech
(86) International application number: PCT/EP2017/000107
(87) International publication number: WO 2017/129370

(56) References cited:
- WO-A1-2007/042034
- WO-A1-2010/079222
- WO-A1-2013/076216
- WO-A1-2015/086467
- WO-A2-2006/037342
- US-A1- 2015 209 318

## Description

### Field of the invention

The present invention relates to a process for the manufacture of a pharmaceutical preparation in the form of a granulate comprising dimethyl fumarate and pharmaceutically acceptable excipients. The invention also relates to a pharmaceutical preparation in the form of granulate obtained by the process of the present invention and to use of the preparation in the treatment of multiple sclerosis.

### Background of the invention

Multiple sclerosis is an autoimmune disease with the activity directed against central nervous system antigens. The disease is characterized by inflammation in parts of the central nervous system, leading to the loss of the myelin, sheathing around neuronal axons (demyelination), axonal loss and the eventual death of neurons, oligodendrocytes and glial cells.

Dimethyl fumarate is used in the treatment of multiple sclerosis. A product in the form of encapsulated enteric coated mini-tablets comprising dimethyl fumarate as the sole active ingredient indicated for the treatment of relapsing remitting multiple sclerosis is marketed by Biogen Idec Ltd. under trade name Tecfidera^{™}.

There are a number of factors that influence the dissolution rate of an active ingredient from the solid dosage form. The method of manufacture, type and concentration of an active substance, type and concentration of excipients, the order of mixing of ingredients during the process, physical parameters of the dosage forms and the compression force used in the process (if applied) all contribute to the dissolution characteristics of the final product.

Drug release from a solid dosage form is usually promoted by disintegrant present in the formulation. Disintegrants are substances or mixtures of substances added to drug formulations which facilitate breakup of solid dosage forms e.g. tablets, granules, pellets into smaller particles. It is commonly observed that a disintegrant used in the granulation process is more effective if added both to the blend before formation of the granule (intragranularly) and to granules prior to compression to the final dosage form (extragranularly). In such case the extragranular portion of the disintegrant facilitates the breakup of the solid dosage form into the granules and the intragranular portion of the disintegrant facilitates further disintegration to release the drug into solution.

WO2007042034 discloses a controlled-release granulate comprising fumaric acid esters obtained by wet granulation and a tablet obtained by compression of the granulate. The tablet can by further coated with enteric coating.

In a wet granulation process the portion of a disintegrant added intragranularly is not as effective as that added extragranularly due to the fact of wetting and drying which reduces disintegration activity of the disintegrant, whereas in a dry granulation process the disintegrant is not exposed to any factors that may reduce its activity therefore it retains its good disintegration characteristics.

Suprisingly it was found that a two-step addition of disintegrant is not necessary to provide the desired dissolution of the active ingredient. In particular, presence of disintegrant in the intragranular phase is not essential for providing an adequate release profile.

### Description of the invention

The invention relates to a process for the manufacture of a pharmaceutical preparation in the form of granulate comprising dimethyl fumarate, which comprises the following steps:
a) blending dimethyl fumarate with pharmaceutically acceptable excipients comprising a filler and/or binder wherein the filler and/or binder is selected from microcrystalline cellulose, mannitol, anhydrous dibasic calcium phosphate and mixtures thereof, a lubricant and optionally an adsorbent;
b) dry granulating the blend obtained in step a);
c) mixing the blend obtained in step b) with pharmaceutically acceptable excipient comprising at least one disintegrant;
d) dry granulating the blend obtained in step c) to obtain a granulate,
with the proviso that pharmaceutically acceptable excipients used in step a) do not comprise disintegrant.

In a preferred embodiment of the invention, dimethyl fumarate is present in the granulate obtained in step d) in the amount from 65 to 95% by weight with respect to the total weight of the granulate.

In another preferred embodiment of the invention, the filler and/or binder is present in the granulate obtained in step d) in the amount from 2 to 25% by weight with respect to the total weight of the granulate.

It should be noted that some of fillers and binders can be used interchangeably as they exhibit both properties.

In a preferred embodiment of the invention, the lubricant is added in a second step to the blend obtained by mixing dimethyl fumarate with pharmaceutically acceptable excipients comprising a filler and/or binder wherein the filler and/or binder is selected from microcrystalline cellulose, mannitol, anhydrous dibasic calcium phosphate and mixtures thereof and optionally an adsorbent.

In another preferred embodiment of the invention, the lubricant is present in the granulate obtained in step d) in the amount from 0.5 to 2% by weight with respect to the total weight of the granulate.

In yet another preferred embodiment of the present invention the lubricant is selected from magnesium stearate, calcium stearate, stearic acid, sodium stearyl fumarate, glyceryl behenate, glyceryl palmitostearate and mixtures thereof.

In a preferred embodiment of the present invention, the adsorbent is present in the granulate obtained in step d) in the amount up to 10% by weight with respect to the total weight of the granulate.

In another preferred embodiment of the present invention, the adsorbent is preferably selected from silicon dioxide, aluminum magnesium silicate and mixtures thereof.

In a preferred embodiment of the invention the disintegrant is present the granulate obtained in step d) in an amount from 3 to 10% by weight with respect to the total weight of the granulate.

In another preferred embodiment of the present invention, the disintegrant is selected from croscarmellose sodium, sodium starch glycolate, crospovidone, low-substituted hydroxypropyl cellulose and mixtures thereof.

In a preferred embodiment of the present invention, the granulate obtained in step d) is further coated with one or more layers of enteric coating.

In another preferred embodiment of the present invention, the enteric coated granulate is further coated by applying a suspension of silicon dioxide, preferably using fluid bed coating method.

In another preferred embodiment of the invention, the process of coating with a suspension of silicon dioxide is performed using aqueous suspension comprising silicon dioxide. Silicon dioxide is preferably the sole solid ingredient forming the suspension.

In a preferred embodiment of the invention, the preparation has three coating layers with two inner coating layers comprising different enteric film-forming polymers and an outer coating formed by fluid bed coating with a suspension comprising silicon dioxide.

A further aspect of the invention relates to a pharmaceutical preparation in the form of granulate obtained by the process as defined above.

In a preferred embodiment of the invention, the granulate of the present invention is filled into capsules or sachets.

Another aspect of the invention relates to a pharmaceutical preparation in the form of granulate obtained by the process as defined above for the treatment of multiple sclerosis.

The following examples are provided to illustrate different aspects and embodiments of the present invention. These examples are not intended in any way to limit the disclosed invention. The skilled person would appreciate that amounts given in tables as a percentage [%] w/w and defining the composition and intermediate and final granulates are equivalent to the amounts of components used for manufacturing the granulates.

### EXAMPLES

### Example 1

Dimethyl fumarate and mannitol were sieved and blended. Then sodium stearyl fumarate was sieved, added to the blend from the previous step and mixed. The blend was dry granulated. Thus obtained granulate was mixed with croscarmellose sodium and dry granulated. The following composition was obtained:

| Ingredient | [%] w/w | mg/caps. |
|---|---|---|
| Intragranularly | | |
| Dimethyl fumarate | 85.71 | 240.00 |
| Mannitol | 9.36 | 26.20 |
| Sodium stearyl fumarate | 1.00 | 2.80 |

| Extragranularly | | |
|---|---|---|
| Croscarmellose sodium | 3.93 | 11.00 |
| TOTAL | 100.00 | 280.00 |

### Example 2

Dimethyl fumarate and mannitol were sieved and blended. Then sodium stearyl fumarate was sieved, added to the blend from the previous step and mixed. Thus obtained blend was dry granulated. The obtained granulate was mixed with croscarmellose sodium and dry granulated. The granulate was fluid bed coated using a suspension of enteric film-forming polymer methacrylic acid - methyl methacrylate copolymer in the ratio of 1:1 by weight (commercially available from Evonik as Eudragit L12.5), talc and triethyl citrate in isopropanol. The enteric coated granulate was then fluid bed coated using aqueous suspension of enteric film-forming polymer methacrylic acid - ethyl acrylate copolymer in the ratio of 1:1 by weight (commercially available from Evonik as Eudragit L30 D-55) and a mixture of triethyl citrate, glycerol monostearate, polysorbate 80. The granulate with two layers of enteric coating was subsequently fluid bed coated using an aqueous suspension of silicon dioxide. Thus obtained enteric coated granulate with an outer silicon dioxide coating was filled into hard gelatin capsules affording the dose of 240 mg per capsule. The following composition was obtained:

| Ingredient | [%] w/w | mg/caps. |
|---|---|---|
| Intragranularly | | |
| Dimethyl fumarate | 46.22 | 240.00 |
| Mannitol | 2.12 | 26.22 |
| Sodium stearyl fumarate | 0.54 | 2.80 |

| Extragranularly | | |
|---|---|---|
| Croscarmellose sodium | 2.59 | 11.00 |

| 1^{st} coating layer | | |
|---|---|---|
| Methacrylic acid - methyl methacrylate copolymer (1:1) | 6.77 | 35.18 |
| Talc | 3.36 | 17.45 |
| Triethyl citrate | 0.65 | 3.38 |

| 2^{nd} coating layer | | |
|---|---|---|
| Methacrylic acid - ethyl acrylate copolymer (1:1) | 29.31 | 152.18 |
| Triethyl citrate | 2.94 | 15.27 |
| Glycerol monostearate | 1.46 | 7.59 |
| Polysorbate 80 | 0.58 | 3.04 |

| 3^{rd} coating layer | | |
|---|---|---|
| Silicon dioxide | 0.99 | 5.14 |
| TOTAL | 100.00 | 519.22 |

### Comparative Example 1:

Dimethyl fumarate, mannitol and croscarmellose sodium were sieved and blended. Then sodium stearyl fumarate was sieved, added to the blend from the previous step and mixed. The blend was dry granulated. The following composition was obtained:

| Ingredient | [%] w/w | mg/caps. |
|---|---|---|
| Intragranularly | | |
| Dimethyl fumarate | 89.22 | 240.00 |
| Mannitol | 5.74 | 15.44 |
| Croscarmellose sodium | 4.00 | 10.76 |
| Sodium stearyl fumarate | 1.04 | 2.80 |
| TOTAL | 100.00 | 269.00 |

### Comparative Example 2:

Dimethyl fumarate, mannitol and croscarmellose sodium were sieved and blended. Then sodium stearyl fumarate was sieved, added to the blend from the previous step and mixed. The blend was dry granulated. Thus obtained granulate was mixed with croscarmellose sodium and dry granulated. The following composition was obtained:

| Ingredient | [%] w/w | mg/caps. |
|---|---|---|
| Intragranularly | | |
| Dimethyl fumarate | 85.71 | 240.00 |
| Mannitol | 3.93 | 11.00 |
| Croscarmellose sodium | 4.55 | 12.75 |
| Sodium stearyl fumarate | 1.00 | 2.80 |

| Extragranularly | | |
|---|---|---|
| Croscarmellose sodium | 4.80 | 13.45 |
| TOTAL | 100.00 | 280.00 |

### Comparison of dissolution

Granulates of Example 1 and Comparative Example 1 and Comparative Example 2 were tested for dissolution in phosphate buffer (pH 6.8) without any pretreatment in a solution of hydrochloric acid. The following results were achieved:

| Tested product | Dissolution in phosphate buffer (pH 6.8) [%] | | | | | | |
|---|---|---|---|---|---|---|---|
| | 5 min. | 10 min. | 15 min. | 20 min. | 30 min. | 45 min. | 60 min. |
| Granulate of Ex. 1 | 46.1 | 80.6 | 89.0 | 92.8 | 92.5 | 94.5 | 97.4 |
| Granulate of Comparative Ex. 1 | 20.1 | 60.6 | 77.5 | 86.5 | 94.8 | 93.7 | 98.6 |
| Granulate of Comparative Ex. 2 | 37.2 | 61.0 | 69.2 | 75.4 | 83.4 | 86.1 | 90.0 |

It was observed that the granulate of Example 1 exhibits better dissolution profile as compared to the granulate of Comparative Example 1 and Comparative Example 2.

In the granulate of Example 1 and the Comparative Example 1 the disintegrant is present in a similar amount. One should expect that release of the active ingredient from granulate of Comparative Example 1 should be more rapid than that of the granulate of Example 1 due to the presence of disintegrant in the core of the granule, which promotes the breakup of the dosage form leading to release of the drug. However, it was observed that after 15 minutes 89% of the dimethyl fumarate from the granulate of Example 1 was released in the phosphate buffer as compared to only 77.5% from the granulate of the Comparative Example 1. After 30 minutes complete release of the active ingredient can be observed in case of both products due to complete disintegration of granulates.

In the Comparative Example 2 the disintegrant in present both in the intragranular and extragranular phase in the total amount of 9.35 % by weight based on the total amount of the granulate. The skilled person would have expected that dissolution profile of the granulate of Comparative Example 2 should be more rapid than that of granulate of Example 1 due to higher content of the disintergrant and also its presence in both the intragranular and extragranular phases. However, the obtained results suggest that about 90 % of the dimethyl fumarate is released after 20 minutes from the granulate of Example 1 whereas for the Comparative Example 2 similar results are achieved after 60 minutes.

Hence, this data suggests that the lack of disintegrant in the intragranular phase of the presented formulation improves dissolution of dimethyl fumarate which likely triggers much faster absorption, resulting in a more rapid onset of the therapeutic effect.

## Claims

1. A process for the manufacture of a pharmaceutical preparation in the form of granulate comprising dimethyl fumarate, which comprises the following steps
a) blending dimethyl fumarate with pharmaceutically acceptable excipients comprising a filler and/or a binder wherein the filler and/or binder is selected from microcrystalline cellulose, mannitol, anhydrous dibasic calcium phosphate and mixtures thereof, a lubricant and optionally an adsorbent;
b) dry granulating the blend obtained in step a);
c) mixing the blend obtained in step b) with a pharmaceutically acceptable excipients comprising at least one disintegrant;
d) dry granulating the blend obtained in step c) to obtain a granulate,
with the proviso that pharmaceutically acceptable excipients used in step a) do not comprise a disintegrant.

2. Process according to claim 1, wherein dimethyl fumarate is present in the granulate obtained in step d) in an amount from 65 to 95 % by weight with respect to the total weight of the granulate.

3. Process according to claims 1-2, wherein the filler and/or binder is present in the granulate obtained in step d) in an amount from 2 to 25 % by weight with respect to the total weight of the granulate.

4. Process according to any of the claims 1-3, wherein the lubricant is present in the granulate obtained in step d) in an amount up to 0.5 to 2 % by weight with respect to the total weight of the granulate.

5. Process according to any of the claims 1-4, wherein the lubricant is selected from magnesium stearate, calcium stearate, stearic acid, sodium stearyl fumarate, glyceryl behenate, glyceryl palmitostearate and mixtures thereof.

6. Process according to any of the claims 1-5, wherein the disintegrant is present in the granulate obtained in step d) in an amount from 3 to 10 % by weight with respect to the total weight of the blend.

7. Process according to any of the claims 1-6, wherein the disintegrant is selected from croscarmellose sodium, sodium starch glycolate, crospovidone, low-substituted hydroxypropyl cellulose and mixtures thereof.

8. Process according to any of the claims 1-7, wherein the granulate obtained in step d) is further coated with one or more layers of enteric coating.

9. Process according to claim8, wherein the enteric coated granulate is further coated with a suspension of silicon dioxide, preferably using fluid bed coating method.

10. Process according to claim9, wherein the suspension of silicon dioxide is an aqueous suspension.

11. An oral pharmaceutical preparation in the form of granulate obtained by the process according to any of claims 1-10.

12. The pharmaceutical preparation according to claim 11, wherein the preparation is filled into capsules or sachets.

13. A pharmaceutical preparation according to claims 11-12 for use in the treatment of multiple sclerosis.

## Patentansprüche

1. Verfahren zum Herstellen einer pharmazeutischen Zubereitung in Form eines Granulats, umfassend Dimethylfumarat, das die folgenden Schritte umfasst:
a) Vermischen von Dimethylfumarat mit pharmazeutisch unbedenklichen Exzipienten, umfassend einen Füllstoff und/oder ein Bindemittel, wobei der Füllstoff und/oder das Bindemittel aus mikrokristalliner Cellulose, Mannit, wasserfreiem dibasischem Calciumphosphat und Gemischen davon ausgewählt ist, einem Schmiermittel und wahlweise einem Adsorptionsmittel;
b) Trockengranulieren der in Schritt a) erhaltenen Mischung;
c) Mischen der in Schritt b) erhaltenen Mischung mit einem pharmazeutisch unbedenklichen Exzipienten, umfassend zumindest ein Zersetzungsmittel;
d) Trockengranulieren der in Schritt c) erhaltenen Mischung, um ein Granulat zu erhalten,
unter der Maßgabe, dass in Schritt a) verwendete pharmazeutisch unbedenkliche Exzipienten kein Zersetzungsmittel umfassen.

2. Verfahren nach Anspruch 1, wobei Dimethylfumarat in dem in Schritt d) erhaltenen Granulat in einer Menge von 65 bis 95 Gew.-% in Bezug auf das Gesamtgewicht des Granulats vorhanden ist.

3. Verfahren nach den Ansprüchen 1 bis 2, wobei der Füllstoff und/oder das Bindemittel in dem in Schritt d) erhaltenen Granulat in einer Menge von 2 bis 25 Gew.-% in Bezug auf das Gesamtgewicht des Granulats vorhanden sind.

4. Verfahren nach einem der Ansprüche 1 bis 3, wobei das Schmiermittel in dem in Schritt d) erhaltenen Granulat in einer Menge von bis zu 0,5 bis 2 Gew.-% in Bezug auf das Gesamtgewicht des Granulats vorhanden ist.

5. Verfahren nach einem der Ansprüche 1 bis 4, wobei das Schmiermittel aus Magnesiumstearat, Calciumstearat, Stearinsäure, Natriumstearylfumarat, Glycerylbehenat, Glycerylpalmitostearat und Gemischen davon ausgewählt ist.

6. Verfahren nach einem der Ansprüche 1 bis 5, wobei das Zersetzungsmittel in dem in Schritt d) erhaltenen Granulat in einer Menge von 3 bis 10 Gew.-% in Bezug auf das Gesamtgewicht der Mischung vorhanden ist.

7. Verfahren nach einem der Ansprüche 1 bis 6, wobei das Zersetzungsmittel aus Croscarmellosenatrium, Natriumstärkeglykolat, Crospovidon, niedrigsubstituierter Hydroxypropylcellulose und Gemischen davon ausgewählt ist.

8. Verfahren nach einem der Ansprüche 1 bis 7, wobei das in Schritt d) erhaltene Granulat ferner mit einer oder mehreren Schichten einer enterischen Beschichtung beschichtet wird.

9. Verfahren nach Anspruch 8, wobei das enterisch beschichtete Granulat ferner mit einer Suspension von Siliciumdioxid beschichtet wird, vorzugsweise unter Verwendung eines Wirbelschichtbeschichtungsverfahrens.

10. Verfahren nach Anspruch 9, wobei die Suspension von Siliciumdioxid eine wässrige Suspension ist.

11. Orale pharmazeutische Zubereitung in Form eines Granulats, das mithilfe des Verfahrens nach einem der Ansprüche 1 bis 10 erhalten wurde.

12. Pharmazeutische Zubereitung nach Anspruch 11, wobei die Zubereitung in Kapseln oder Beutelchen gefüllt wird.

13. Pharmazeutische Zubereitung nach den Ansprüchen 11 bis 12 zur Verwendung bei der Behandlung von multipler Sklerose.

## Revendications

1. Procédé de fabrication d'une préparation pharmaceutique sous la forme de granulé comprenant du fumarate de diméthyle, qui comprend les étapes suivantes
a) le mélange du fumarate de diméthyle avec des excipients pharmaceutiquement acceptables comprenant une charge et/ou un liant, dans lequel la charge et/ou le liant sont choisis parmi la cellulose microcristalline, le mannitol, le phosphate de calcium dibasique anhydre et des mélanges de ceux-ci, un lubrifiant et éventuellement un absorbant ;
b) la granulation à sec du mélange obtenu à l'étape a) ;
c) le mélange du mélange obtenu à l'étape b) avec un excipient pharmaceutiquement acceptable comprenant au moins un désintégrant ;
d) la granulation à sec du mélange obtenu à l'étape c) pour obtenir un granulé,
à condition que des excipients pharmaceutiquement acceptables utilisés à l'étape a) ne comprennent pas de désintégrant.

2. Procédé selon la revendication 1, dans lequel le fumarate de diméthyle est présent dans le granulé obtenu à l'étape d) en une quantité de 65 à 95 % en poids par rapport au poids total du granulé.

3. Procédé selon les revendications 1 et 2, dans lequel la charge et/ou le liant sont présents dans le granulé obtenu à l'étape d) en une quantité de 2 à 25 % en poids par rapport au poids total du granulé.

4. Procédé selon l'une quelconque des revendications 1 à 3, dans lequel le lubrifiant est présent dans le granulé obtenu à l'étape d) en une quantité allant jusqu'à 0,5 à 2 % en poids par rapport au poids total du granulé.

5. Procédé selon l'une quelconque des revendications 1 à 4, dans lequel le lubrifiant est choisi parmi le stéarate de magnésium, le stéarate de calcium, l'acide stéarique, le stéarylfumarate de sodium, le béhénate de glycéryle, le palmitostéarate de glycéryle et des mélanges de ceux-ci.

6. Procédé selon l'une quelconque des revendications 1 à 5, dans lequel le désintégrant est présent dans le granulé obtenu à l'étape d) en une quantité de 3 à 10 % en poids par rapport au poids total du mélange.

7. Procédé selon l'une quelconque des revendications 1 à 6, dans lequel le désintégrant est choisi parmi la croscarmellose sodique, le glycolate d'amidon sodique, la crospovidone, l'hydroxypropylcellulose faiblement substituée et les mélanges de ceux-ci.

8. Procédé selon l'une quelconque des revendications 1 à 7, dans lequel le granulé obtenu à l'étape d) est en outre enrobé d'une ou de plusieurs couches d'enrobage entérique.

9. Procédé selon la revendication 8, dans lequel le granulé à enrobage entérique est en outre enrobé d'une suspension de dioxyde de silicium, de préférence à l'aide d'un procédé d'enrobage en lit fluidisé.

10. Procédé selon la revendication 9, dans lequel la suspension de dioxyde de silicium est une suspension aqueuse.

11. Préparation pharmaceutique orale sous la forme de granulé obtenue par le procédé selon l'une quelconque des revendications 1 à 10.

12. Préparation pharmaceutique selon la revendication 11, dans laquelle la préparation est conditionnée dans des capsules ou des sachets.

13. Préparation pharmaceutique selon les revendications 11 et 12, destinée à être utilisée dans le traitement de la sclérose en plaques.
